# EUROPEAN PATENT SPECIFICATION

(11) **EP 3 534 796 B1**
(45) Date of publication and mention of the grant of the patent: **23.03.2022**
(21) Application number: 17804945.8
(22) Date of filing: 31.10.2017
(51) Int. Cl.: A61B 6/00, A61B 6/04

(54) **APPARATUS FOR X-RAY BONE DENSITOMETRY**
VORRICHTUNG ZUR KNOCHENDICHTEMESSUNG MITTELS RÖNTGEN
APPAREIL POUR DENSITOMÉTRIE OSSEUSE PAR RAYONS X

(30) Priority: 02.11.2016 IT 201600109999
(43) Date of publication of application: 11.09.2019
(73) Proprietor: Eurotec Medical Systems S.r.l., 24020 Scanzorosciate, Bergamo (IT)
(72) Inventor: ASSOLARI, Giovanni Fiorenzo, 24060 Torre De' Roveri (BG) (IT)
(74) Representative: Gualeni, Nadia
(86) International application number: PCT/IB2017/056749
(87) International publication number: WO 2018/083587

(56) References cited:
- WO-A1-88/08688
- WO-A1-2006/058420
- WO-A1-2013/157416

## Description

The present invention relates to an apparatus for bone densitometry, in particular, an apparatus for bone densitometry which uses a DEXA method.

The analysis of bone densitometry allows the determination of bone density in the districts at greater risk of fracture (such as the spine, femur and forearm, as well as the whole body) and supports the physician in diagnosing osteoporosis, in fracture risk assessment and in monitoring the response to therapy.

One of the most accurate and reliable techniques for the assessment of bone densitometry, with insignificant radiation doses to the patient, is DEXA (Dual Energy X-Ray Absorptiometry).

This method is based on the assessment of bone mineral density (BMD) and provides two very important indicators: the T-Score (deviation of the mineral density compared to the normal value at about 30 years age and the Z-Score (deviation of the mineral density compared to subjects of the same age). The examinations carried out by the machine also allow assessing the body composition (lean mass, fat mass and bone mass) in certain clinical conditions and setting and monitoring the most suitable dietary treatments.

Apparatuses for bone densitometry are known in the field; examples are described in documents WO 2013/157416 and EP0761166.

The apparatus described in EP0761166 comprises a patient-carrier table and a movable arm provided with an RX generator-detector assembly. The arm is movable longitudinally by means of a movement system (guide rails and belts) resting directly on the ground. Therefore, the space below the patient-carrier table is completely occupied by the movement system of the movable arm, making it difficult to clean the area below it.

WO 2013/157416 describes an apparatus comprising a patient-carrier table and a longitudinally movable arm, which is supported by the patient-carrier table.

Both of the known devices comprise an extremely large number of components, which makes the assembly, installation and maintenance of the apparatus complex.

The object of the present invention is to propose an apparatus for bone densitometry with DEXA method which allows obviating the drawbacks mentioned above, in particular that has a very simple structure that allows having free space below the patient-carrier table, while being able to withstand high weight and height patients.

Said objects are achieved with an apparatus for bone densitometry according to the following claims.

The details and the advantages of the apparatus for bone densitometry according to the present invention will be apparent from the following description of preferred embodiments thereof, provided purely by way of a nonlimiting example, with reference to the accompanying drawings, in which:
Figure 1 is an overall view of an apparatus for bone densitometry according to the invention, fitted with a patient-carrier frame and a movable arm fitted with an RX generator-detector assembly;
Figure 2 is a front view of the apparatus in figure 1;
Figures 3 and 4 show both the lower portion of the patient-carrier frame in figure 1, in an axonometric view and a bottom view, respectively;

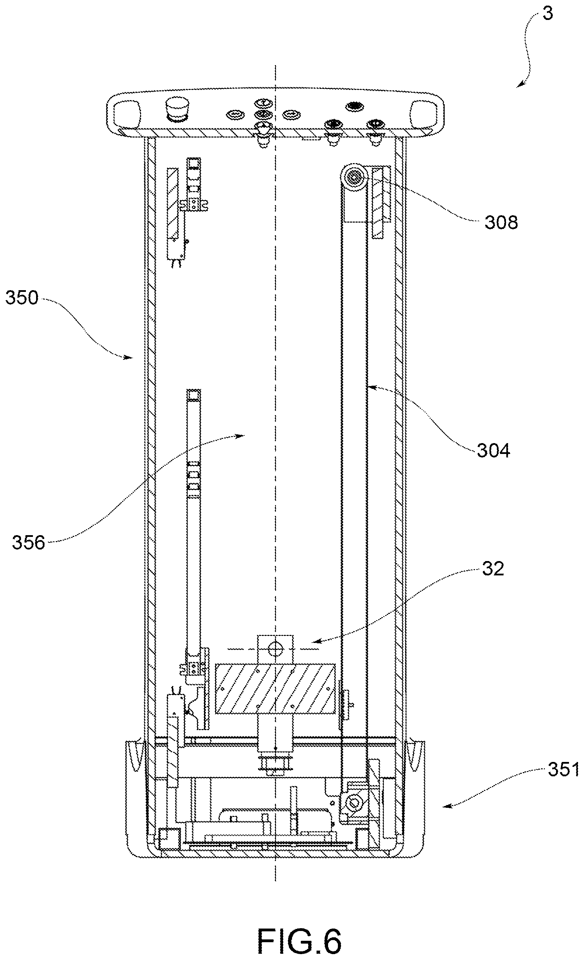

Figures 5 and 6 show both a section of the movable arm in figure 1, in a side view and a bottom view, respectively;
Figure 7 shows a detail of the movement system of the movable arm;
Figure 8 shows the outer shell of the movable arm;

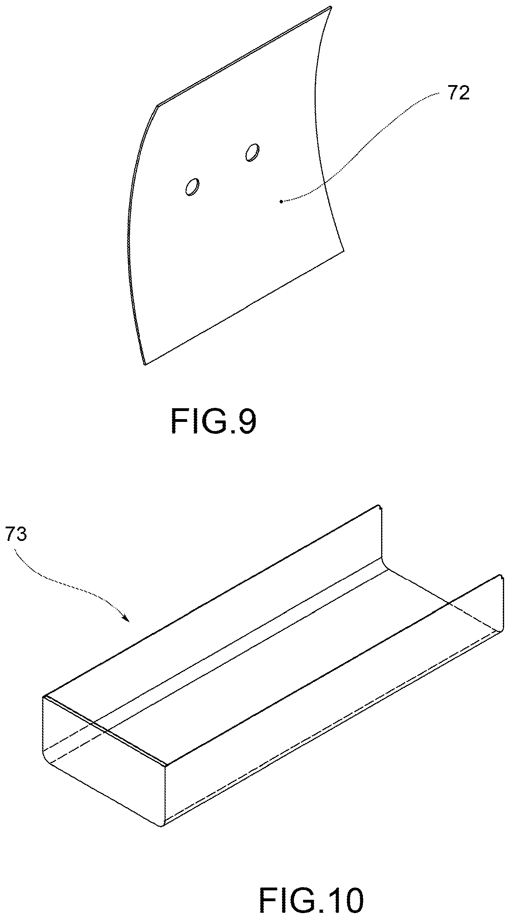

Figures 9 and 10 show further elements of completion, or casing, of the outer shell of the movable arm, respectively a front casing and a lower casing.

The apparatus for bone densitometry, indicated in the above figures with reference numeral 1, consists of mechanical, electronic and software parts integrated according to the technical regulations and to legislation in the field of medical devices.

The apparatus for bone densitometry 1 of the present invention uses a DEXA (Dual Energy X-Ray Absorptiometry) method.

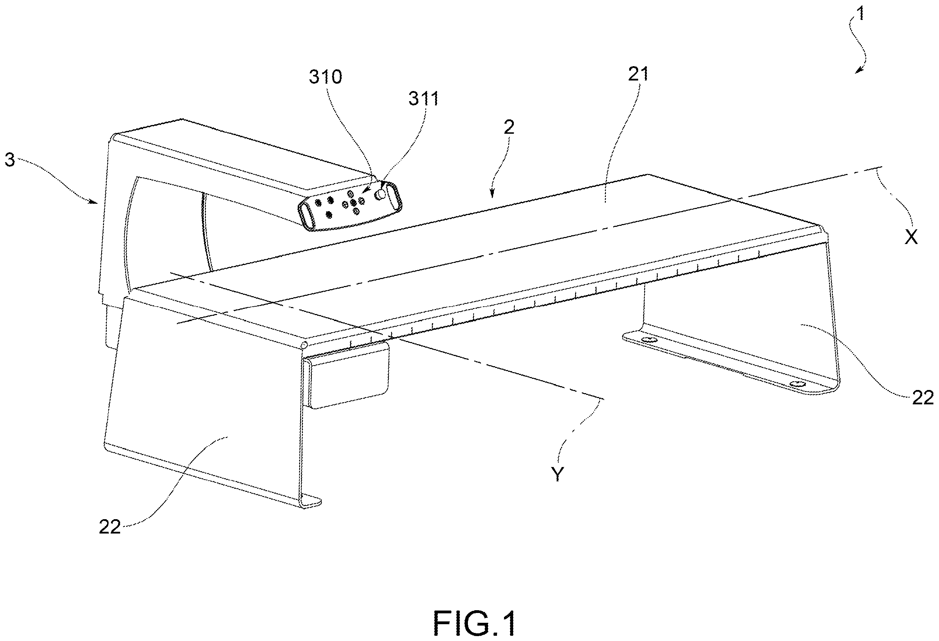

As shown in figure 1, the apparatus for bone densitometry 1 comprises a patient-carrier frame 2 which supports an arm 3 movable along frame 2 and provided with a generator-detector assembly.

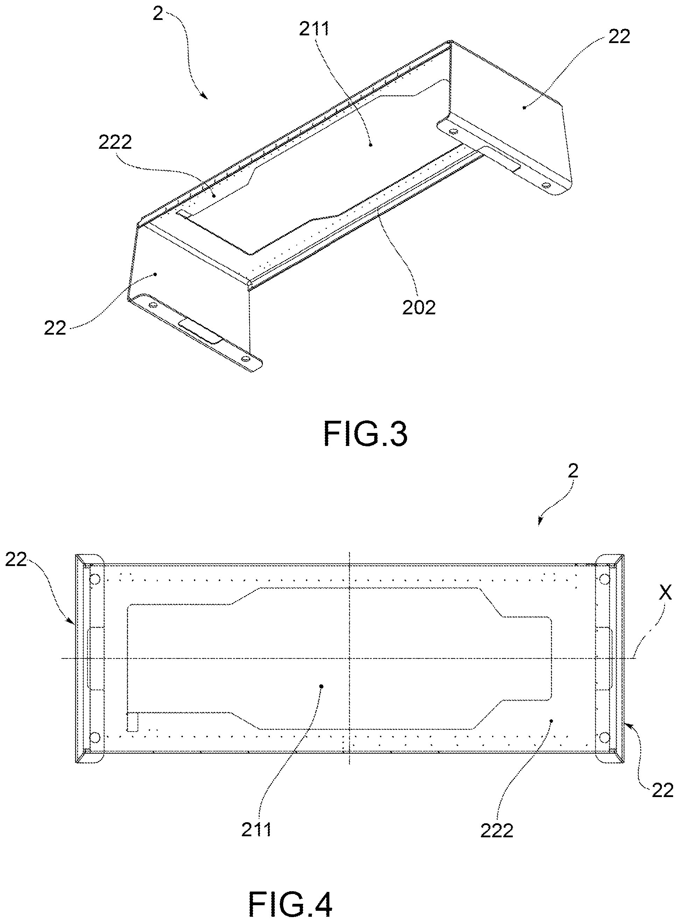

As shown also in figure 3, the apparatus for bone densitometry 1 is characterised by the presence of a monocoque patient-carrier frame 2 adapted to completely support, at the patient's support surface (i.e. the horizontal plane) the movable arm 3 fitted with the generator-detector assembly. Therefore, frame 2 acts both as a support for the patient and for arm 3.

The term "monocoque" means a single rigid structure with bearing function.

Therefore, in the apparatus for bone densitometry 1, the movable arm 3 is supported directly by frame 1 at the support surface 21 on which the patient lies. Such a solution allows having a completely free space below frame 2.

Advantageously, such a solution simplifies the cleaning of the area below the patient-carrier table or frame 2, thus improving the hygiene in the room where the apparatus for bone densitometry 1 is installed.

Advantageously, moreover, such a solution greatly reduces the number of the constituent parts of the apparatus for bone densitometry 1, almost completely eliminating the need for covers. Moreover, such a solution allows achieving a significant ease of transport and installation, as well as easier access for maintenance.

The apparatus for bone densitometry 1 acts as a planar scanner, being formed by a fixed stand (patient-carrier frame 2) and by an arm 3 which moves along the longitudinal direction X, from the feet to the head of the patient.

Arm 3 is fitted with a generator-detector assembly, mechanically aligned so that the collimated beam F of X-rays produced by generator 31 perfectly centres detector 32.

Generator 31 and detector 32 integrally translate both in the longitudinal direction X, by means of a longitudinal movement system 201, and in a transverse direction Y, by means of a transverse movement system 301. During the movement along axis Y, one or more lines of an image are acquired by attenuation of X-rays, while the translation along axis X serves for positioning at the next line. Through the longitudinal and transverse movement of the generator-detector assembly, an X-Y scan of a certain region of interest of the patient is achieved.

The apparatus for bone densitometry 1 further comprises a control unit, which implements the acquisition of the signals coming from detector 32, the control of x-ray emission from generator 31, and the control of the generator-detector assembly movement. The control unit also comprises software for bone densitometry.

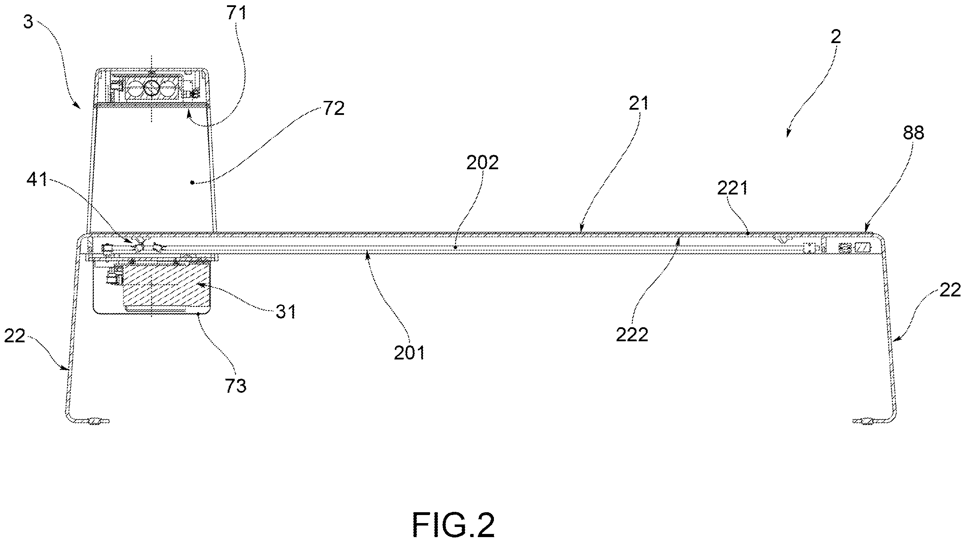

An exemplary embodiment of the apparatus for bone densitometry 1 will now be described in detail. As shown in figure 2, frame 2 comprises a support surface 21, on which the patient lies, supported by legs 22.

The support surface 21 comprises an upper side 221 on which the patient lies, and a lower side 222, opposite the upper side 221, to which the longitudinal movement system 201 of arm 3 is attached.

The patient-carrier frame 2 is made starting from a single sheet of aluminium, folded to form a patient-carrier table.

In particular, frame 2 consists of a single sheet of folded aluminium. In particular, frame 2 consists of a single sheet of aluminium having a thickness of 10 mm. Advantageously, such a solution ensures structural strength: in fact, frame 2 allows a deformation not exceeding 1÷2 mm when loaded with a weight of 100 kg at the centre of the support surface 21.

Preferably, the support surface 21 of the patient comprises a scanning area 211 at which the patient lies. Preferably, the scanning area 211 has a length of about 200 cm and a width of about 70 cm.

The scanning area 211 is obtained by reducing the thickness of frame 2 (for example, originally 10 mm) to 4 mm. Such a solution allows carrying out a filtration of the X-ray beam necessary to eliminate the lower energy component. Frame 2 then comprises a filtering portion for X-rays, formed on the scanning area 211.

The apparatus for bone densitometry 1 is therefore also characterised in that frame 2 itself acts as a filter for the X-rays

In a further example not forming part of the present invention, frame 2 is made starting from a single sheet of carbon fibre, folded to form a patient-carrier table.

The dimensions of frame 2 are such as to enable scanning the whole body of the patient, even for patients with high height and high weight.

For example, the support surface 21 has a length of about 2.5 metres and a width of about 1 metre.

Preferably, in order to facilitate the positioning of the patient on frame 2, the support surface 21 is placed at a height of about 60 cm from the floor surface.

The longitudinal movement system 201 of arm 3 is mounted on frame 2, at the lower side 222 of the support surface 21.

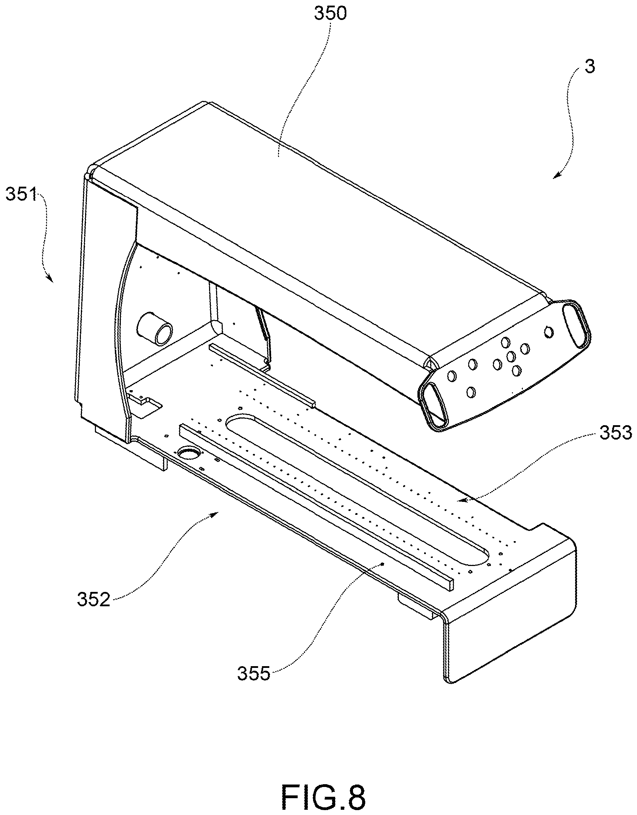

Preferably, as shown in figure 8, arm 3 is also monocoque.

In a preferred exemplary embodiment, arm 3 is made starting by folding a single sheet of metal, preferably aluminium with a thickness of 10 mm. Advantageously, arm 3 is considerably light, for example not exceeding 40 kg in weight.

Preferably, arm 3 is "U"-shaped.

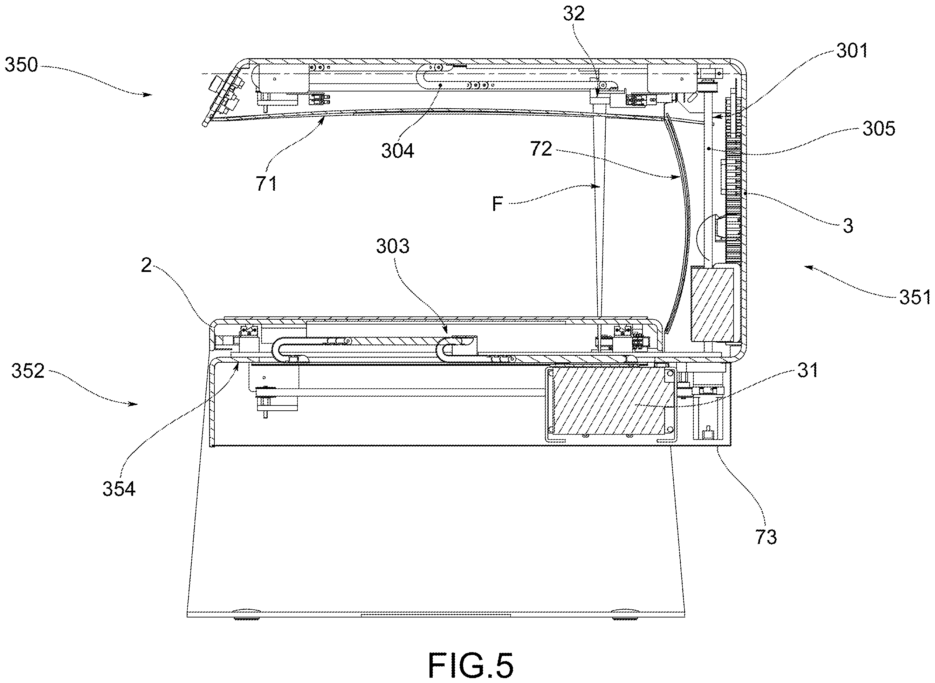

As shown in figure 5, arm 3 comprises an upper portion 350, an intermediate portion 351, and a lower portion 352.

Arm 3 comprises all the active parts of the machine: X-ray generator 31 and X-ray detector 32, a pointer for the positioning of the patient, control electronics, motors 306, 316 for the longitudinal and transverse movements of arm 3, as well as, for example, a keyboard 310 for movement, a button 311 for emergency stop, buttons for movement and laser starting and signalling LEDs.

Preferably, arm 3 comprises, at the upper portion 350, a support plate 356 for detector 32 and for the pointer. The pointer is a low-power semiconductor laser, which acts as a reference point for the purposes of patient centring.

Preferably, arm 3 comprises, at the upper portion 350: detector 32, a pointer, keyboard 310, emergency button 311, buttons for movement and laser starting and signalling LEDs.

Preferably, arm 3 comprises, at the central portion 351: control and communication electronics, power supplies, motor drivers.

Preferably, arm 3 comprises, at the lower portion 352: generator 31, motors 306, 316 for the longitudinal and transverse movements.

Preferably, arm 3 comprises, at the lower portion 352, a support plate 353 for generator 31. In particular, generator 3 is hung on the lower side 354 of the support plate 353. The transverse movement system 301, and in particular the runners of guide 303 for moving generator 31, is instead attached at the upper side 355 of the support plate 353.

As mentioned above, the generator-detector assembly translate both in the longitudinal direction X, by means of the longitudinal movement system 201, and in a transverse direction Y, by means of the transverse movement system 301. The transmission of both the longitudinal (axis X) and transverse (axis Y) movement consists of a set of linear guides, carriages, belts, pulleys and bearings. Both motor 306 for the transverse movement and motor 316 for the longitudinal movement are mounted on arm 3.

The whole arm 3 is movable longitudinally along the patient-carrier frame 1 (i.e. along axis X) by means of the longitudinal movement system 201 supported directly by frame 1.

The longitudinal movement (axis X) takes place by dragging over a fixed toothed belt. Motor 316 transmits the movement through a system of belts and pulleys.

The longitudinal movement system 201 comprises a toothed belt drive, coupled to a pair of guides 202 mounted on the lower side 222 of the support surface 21. In this way, arm 3 moves suspended hung on frame 21, leaving completely free access to the underlying floor.

The linear guides 202 fitted with suitable carriages, support arm 3 and allow the translation thereof along axis X.

The mechanical connection between arm 3 and frame 2 is achieved by means of carriages 41.

The transverse movement system 301 of the generator-detector assembly is mounted on arm 3. Once the alignment has been adjusted, generator 31 and detector 32 move integrally and maintain the alignment along axis Y.

The movements of detector 32 and generator 31 are made integral precisely through the transverse movement system, in such a way that generator 31 and detector 32 achieve a perfect mechanical alignment with respect to the direction of the ray beam F.

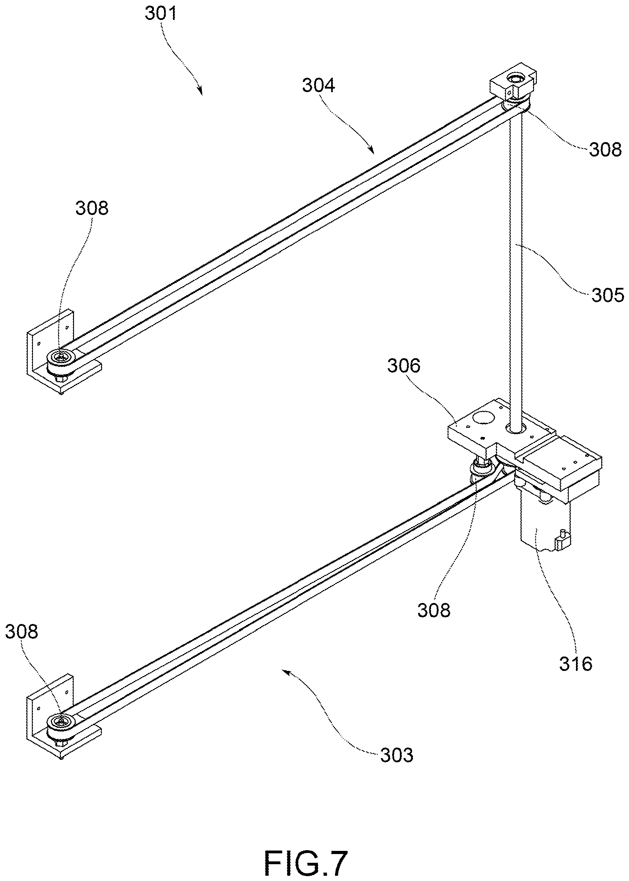

As shown in figure 7, the transverse movement system 301 comprises a belt 303 for the movement of generator 31, placed in the lower part of arm 3, and a belt 304 for the movement of detector 32, placed in the upper part of arm 3.

Each belt 303, 304, preferably toothed, is wound around a respective pair of pulleys 308.

The transverse movement system 301 further comprises a drive rod 305, or a drive belt, for the transmission of motion from motor 306 to belts 303, 304, and for the correct synchronisation of the movement of generator 31 of detector 32.

The apparatus for bone densitometry 1 is also characterised in that the cover elements (also called casing) of the active parts of the machine housed in arm 3 are reduced to the minimum.

In particular, the cover elements comprise an upper casing 71, a central casing 72, and a lower casing 73.

The upper casing 71 is placed to close the upper portion 350 of arm 3 and to cover detector 32 by the whole stroke of movement Y. The upper casing 71 is preferably made in the form of a sheet of Plexiglas and is inserted into dedicated guides provided in the upper portion 350.

The central casing 72 is placed to close the central portion 351 and to cover the electronics mounted on arm 3. The central casing 72 is preferably made of aluminium.

The lower casing 73 is placed to close the lower portion 352 and to cover generator 31 and guide 303.

Preferably, the apparatus for bone densitometry 1 further comprises accessories which allow the patient to be subjected to the examination in comfortable conditions.

Preferably, the support surface 21 on which the patient lies is covered with a mat 88 of X-ray transparent material.

Preferably, the surface of mat 88 is washable and waterproof, in order to limit the risk of microbial contamination due to the promiscuity of use with potentially infected patients.

Preferably, mat 88 is made with a fireproof and anti-allergic material.

Preferably, a brass collimator is mounted on the outlet mouth of generator 31. The collimator is thick enough to ensure the total shielding of the rays outside of the collimated beam (at least 20 mm). The geometry of the collimator ensures, in addition to the collimation, also shielding from X-rays outside the primary beam.

Preferably, a brass collimator (10 mm) diameter is also mounted on the detector 32 side. The collimation on the detector side serves to minimise the effect of any scattered radiation; it constitutes a means to adjust the efficiency of the detector and the system spatial resolution.

Advantageously, all the constituent elements of the apparatus for bone densitometry 1 are made in one piece, possibly folded, without the need for screws: monocoque frame 2, monobloc arm 3, casings 71, 72, 73.

Innovatively, an apparatus for bone densitometry according to the present invention has a very simple structure that allows having free space below the patient-carrier table, while being able to withstand high weight and height patients.

To summarise, therefore, the innovative and advantageous aspects of the apparatus for bone densitometry 1 according to the present invention:
- monocoque patient-carrier frame 2 adapted to support, at the support surface 21 for the patient, the movable arm 3 fitted with a generator-detector assembly: area under the table completely clear and easy to clean; easy accessibility for patients with locomotory problems (e.g. wheelchair) who can be positioned close to the table, getting on and off independently;
- apparatus for bone densitometry 1 consisting of an extremely small number of components: monobloc frame 2, monobloc arm 3, only three casings 71, 72, 73: ease of transport, mounting simplicity, rapidity in the possible need to move, installation and maintenance;
- patient-carrier frame 2 fitted with a filtering portion for X-rays, consisting of the scanning area 211: possibility of making a metal frame (aluminium alloy) capable of supporting patients of considerable height and weight (even up to 410 kg and 2 m).

A man skilled in the art may make several changes and adjustments to the apparatus for bone densitometry described above in order to meet specific and incidental needs, all falling within the scope of protection defined in the following claims.

## Claims

1. Apparatus for bone densitometry (1), comprising:
- a patient-carrier frame (2) with a support surface (21) comprising a scanning area and being configured to support a lying patient;
- an arm (3) movable with respect to the patient-carrier frame (2) in a longitudinal direction (X) by means of a longitudinal movement system (201), from the feet to the head of the patient lying on the support surface, and comprising a generator-detector assembly comprising an X-ray generator (31) and an X-ray detector (32), which are mechanically aligned so that the collimated beam (F) of X-rays produced by the generator (31) centres the X-ray detector (32);
- a control unit which is configured to implement Dual Energy X-ray Absorptiometry (DEXA) ;
**characterised in that** the patient-carrier frame (2) is a monocoque (2), that is a single rigid structure with bearing function, and consists of a single sheet of aluminium ,which has been folded to form the patient-carrier table, and which fully supports the arm (3) ,**in that** the longitudinal movement system is attached to a lower side of the support surface, which is opposite to the side on which the patient lies, in order to leave the space underneath the patient-carrier frame (2) free, and **in that** the scanning area (211) is formed by reducing the thickness of the frame (2) to create a filtering portion (211) for the X-rays.

2. Apparatus for bone densitometry (1), according to claim 1, wherein the scanning area (211) has a length of about 200 cm and a width of about 70 cm.

3. Apparatus for bone densitometry (1), according to claim 1 or 2, wherein the frame (2) is made starting from a single sheet of aluminium of 10 mm and in correspondence with the scanning area (211) the thickness of the frame is reduced to 4 mm.

4. Apparatus for bone densitometry (1), according to any of the preceding claims, wherein the generator-detector assembly is configured to translate with respect to the frame (2) in a transverse direction (Y), by means of a transverse movement system (301) so that a scan (X-Y) of a given region of interest of the patient can be performed.

5. Apparatus for bone densitometry (1), according to any of the preceding claims, wherein the longitudinal movement system (201) comprises a pair of guides (202) mounted on the lower side (222) of the support plane (21) and a toothed belt drive coupled to said pair of guides.

6. Apparatus for bone densitometry (1), according to claim 4 or 5, wherein the transverse movement system (301) comprises a belt (303) for the movement of the generator (31), placed in a lower part of the arm (3), and a belt (304) for the movement of the X-ray detector (32), placed in an upper part of the arm (3), connected by a drive belt (305) for the transmission of the movement from a motor (306) to the belts (303, 304) and for the correct synchronisation of the movement of the X-ray generator (31) and of the X-ray detector (32).

7. Apparatus for bone densitometry (1), according to any of the preceding claims, wherein the arm (3) is monocoque and houses active parts of the apparatus and further comprises casings (71, 72, 73) for the active parts of the apparatus .

## Patentansprüche

1. Vorrichtung zur Knochendichtemessung (1), umfassend:
- einen Patiententrägerrahmen (2) mit einer Stütz- bzw. Auflagefläche (21), die einen Scanbereich umfasst und konfiguriert ist, einen liegenden Patienten zu stützen;
- einen Arm (3), der bezüglich des Patiententrägerrahmens (2) in einer Längsrichtung (X) mittels eines Längsbewegungssystems (201) von den Füßen zum dem Kopf des auf der Stützfläche liegenden Patienten beweglich ist, und umfassend eine Generator-Detektor-Anordnung, die einen Röntgenstrahlengenerator (31) und einen Röntgenstrahlendetektor (32) umfasst, die mechanisch so ausgerichtet sind, dass der kollimierte Strahl (F) von Röntgenstrahlen, die von dem Generator (31) erzeugt werden, den Röntgendetektor (32) zentriert;
- eine Steuer- bzw. Regeleinheit, die konfiguriert ist, Dual Energy X-ray Absorptiometry bzw. Doppelröntgen-Absorptiometrie (DEXA) zu implementieren;
**dadurch gekennzeichnet, dass** der Patiententrägerrahmen (2) ein Monocoque (2) ist, das eine einzelne starre Struktur mit tragender Funktion ist, und aus einem einzelnen Aluminiumblech besteht, das dahingehend gefaltet wurde, den Patiententrägertisch zu bilden, und das den Arm (3) vollständig stützt, indem das Längsbewegungssystem an einer unteren Seite der Stützfläche angebracht ist, die der Seite gegenüberliegt bzw. entgegengesetzt ist, auf welcher der Patient liegt, um den Raum unterhalb des Patiententrägerrahmens (2) frei zu lassen,
und dadurch, dass der Scanbereich (211) durch Verringern der Dicke des Rahmens (2) gebildet ist bzw. wird, um einen Filterabschnitt (211) für die Röntgenstrahlen zu schaffen.

2. Vorrichtung zur Knochendichtemessung (1) nach Anspruch 1, wobei der Scanbereich (211) eine Länge von etwa 200 cm und eine Breite von etwa 70 cm aufweist.

3. Vorrichtung zur Knochendichtemessung (1) nach Anspruch 1 oder 2, wobei der Rahmen (2) ausgehend von einem einzelnen Aluminiumblech von 10 mm hergestellt ist und entsprechend dem Scanbereich (211) die Dicke des Rahmens auf 4 mm verringert ist.

4. Vorrichtung zur Knochendichtemessung (1) nach einem der vorhergehenden Ansprüche, wobei die Generator-Detektor-Anordnung konfiguriert ist, sich bezüglich des Rahmens (2) in einer Querrichtung (Y) mittels eines Querbewegungssystems (301) zu verschieben, so dass ein Scan (X-Y) einer gegebenen, interessierenden Region des Patienten durchgeführt werden kann.

5. Vorrichtung zur Knochendichtemessung (1) nach einem der vorhergehenden Ansprüche, wobei das Längsbewegungssystem (201) ein Paar Führungen (202), die an der unteren Seite (222) der Stützebene (21) angebracht sind, und einen mit dem Paar Führungen gekoppelten Zahnriemenantrieb umfasst.

6. Vorrichtung zur Knochendichtemessung (1) nach Anspruch 4 oder 5, wobei das Querbewegungssystem (301) einen Riemen (303) für die Bewegung des Generators (31), der in einem unteren Teil des Arms (3) platziert ist, und einen Riemen (304) für die Bewegung des Röntgendetektors (32) umfasst, der in einem oberen Teil des Arms (3) platziert ist, und zwar verbunden durch einen Antriebsriemen (305) für die Übertragung der Bewegung von einem Motor (306) auf die Riemen (303, 304) und für die korrekten Synchronisation der Bewegung des Röntgengenerators (31) und des Röntgendetektors (32).

7. Vorrichtung zur Knochendichtemessung (1) nach einem der vorhergehenden Ansprüche, wobei der Arm (3) monocoque ist und aktive Teile der Vorrichtung aufnimmt, und ferner Gehäuse (71, 72, 73) für die aktiven Teile der Vorrichtung umfasst.

## Revendications

1. Appareil pour densitométrie osseuse (1), comprenant :
- un cadre porteur de patient (2) ayant une surface de support (21) comportant une zone de balayage et étant configuré pour supporter un patient couché,
- un bras (3), mobile par rapport au cadre porteur de patient (2) dans une direction longitudinale (X) à l'aide d'un système de déplacement longitudinal (201) des pieds à la tête du patient couché sur la surface de support, et comprenant un ensemble générateur-détecteur avec un générateur de rayons X (31) et un détecteur de rayons X (32) qui sont mécaniquement alignés, si bien que le rayon collimaté (F) de rayons X engendré par le générateur (31), vise le centre du détecteur de rayons X (32),
- une unité de commande qui est configurée pour mettre en œuvre l'absorptiométrie biénergétique à rayons X (DEXA),
**caractérisé en ce que** le cadre porteur de patient (2) est une monocoque (2), c'est-à-dire une structure rigide unique avec une fonction de portage, et est constitué d'une feuille unique d'aluminium qui a été pliée pour former la table porteur de patient et qui porte entièrement le bras (3), **en ce que** le système de déplacement longitudinal est attaché à un côté inférieur de la surface de support qui est opposée au côté sur lequel le patient est couché, afin de laisser l'espace en-dessous du cadre porteur de patient (2) libre, et **en ce que** la zone de balayage (211) est formée en réduisant l'épaisseur du cadre (2) pour créer une partie de filtration (211) pour les rayons X.

2. Appareil pour densitométrie osseuse (1) selon la revendication 1, **caractérisé en ce que** la zone de balayage (211) présente une longueur d'environ 200 cm et une largeur d'environ 70 cm.

3. Appareil pour densitométrie osseuse (1) selon la revendication 1 ou 2, **caractérisé en ce que** le cadre (2) est réalisé à partir d'une feuille unique d'aluminium de 10 mm et, correspondant à la zone de balayage (211), l'épaisseur du cadre est réduite à 4 mm.

4. Appareil pour densitométrie osseuse (1) selon l'une des revendications précédentes, **caractérisé en ce que** l'ensemble générateur-détecteur est configuré pour se déplacer par rapport au cadre (2) dans une direction transversale (Y) à l'aide d'un système de déplacement transversal (301), si bien qu'un balayage (X-Y) d'une zone d'intérêt donnée du patient puisse être effectué.

5. Appareil pour densitométrie osseuse (1) selon l'une des revendications précédentes, **caractérisé en ce que** le système de déplacement longitudinal (201) comprend une paire de guides (202) montée sur le côté inférieur (222) du plan de support (21) et un entraînement à courroie dentée accouplé à la paire de guides.

6. Appareil pour densitométrie osseuse (1) selon la revendication 4 ou 5, **caractérisé en ce que** le système de déplacement transversal (301) comprend une courroie (303) pour le déplacement du générateur (31), placée dans une partie inférieure du bras (3), et une courroie (304) pour le mouvement du détecteur de rayons X (32), placée dans une partie supérieure du bras (3), reliées par une courroie d'entraînement (305) pour la transmission du déplacement, d'un moteur (306) aux courroies (303, 304) et pour la synchronisation correcte du déplacement du générateur de rayons X (31) et du détecteur de rayons X (32).

7. Appareil pour densitométrie osseuse (1) selon l'une des revendications précédentes, **caractérisé en ce que** le bras (3) est monocoque et loge des parties actives de l'appareil et comprend en outre des boîtiers (71, 72, 73) pour les parties actives de l'appareil.
